# EUROPEAN PATENT APPLICATION

(11) **EP 4 741 005 A1**
(43) Date of publication of application: **13.05.2026**
(21) Application number: 25213307.9
(22) Date of filing: 04.11.2025
(51) Int. Cl.: A61N 5/10

(54) **METHOD AND APPARATUS FOR OPTIMIZING A RADIATION TREATMENT PLAN**

(30) Priority: 07.11.2024 US 202418939679
(71) Applicant: Siemens Healthineers International AG, 6312 Steinhausen (CH)
(72) Inventor: KUUSELA, Esa, 02320 Espoo (FI)
(74) Representative: Mathisen & Macara LLP

(57) **Abstract**

A control circuit 101 identifies 301 a plurality of radiation treatment arclets for a given radiation treatment platform and then identifies 302, for each of the plurality of radiation treatment arclets, a corresponding isocenter location within the patient 104. The foregoing can result in locating the platform isocenter at different locations within the patient during the course of a radiation treatment session for different ones of the arclets. A radiation treatment plan is optimized 303 as a function of the plurality of arclets and their corresponding isocenter locations. By one approach, these teachings will include automatically dividing at least one initial template arc into at least two radiation treatment arclets. By one approach, identifying the corresponding isocenter locations for each of the plurality of radiation treatment arclets can comprise identifying the corresponding isocenter locations to maintain close proximity of a radiation treatment platform collimator to an exterior surface of the patient.

## Description

### TECHNICAL FIELD

These teachings relate generally to treating a patient's planning target volume with energy pursuant to an energy-based treatment plan and more particularly to optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

In at least some application settings, dose conformality can be a useful measure of the quality of a given radiation treatment plan. Radiation treatment platform physical properties can play a role with respect to dose conformality results. As one example, the penumbra of a beamlet achieved by a multi-leaf collimator can be a limiting factor impacting achievable dose conformality. The width of beamlet penumbra is dependent upon numerous design and implementation details of a multi-leaf collimator, such as leaf tip shape and the elevation of the multi-leaf collimator with respect to the platform's isocenter. The latter can affect penumbra width indirectly by contributing to the distance that the beamlet travels through air before reaching the patient (and ultimately the target region). The applicant has determined that this air-based travel can cause scatter. In addition, this distance can itself cause an increase in the penumbra because the beamlets are often divergent.

### SUMMARY

In one aspect the present invention provides a method as defined in claim 1. The method may be for use with a radiation treatment platform having a corresponding platform isocenter and a movement capability of moving a patient with respect to the platform isocenter while undergoing radiation treatment by the radiation treatment platform. The method may further comprise administering therapeutic radiation to the patient via the radiation treatment platform in a treatment session using the optimized radiation treatment plan, which may optionally include automatically adjusting, during the treatment session, a distance between a surface of the patient and a part of the radiation treatment platform to avoid a collision therebetween. Other optional features are specified in the dependent claims.

In another aspect the present invention provides an apparatus as defined in claim 7. Optional features are specified in the dependent claims.

### BRIEF DESCRIPTION OF DRAWINGS

The above needs are at least partially met through provision of the method and apparatus for optimizing a radiation treatment plan described in the following detailed description, particularly when studied in conjunction with the drawings, wherein:
FIG. 1 comprises a block diagram as configured in accordance with various embodiments of these teachings;
FIG. 2 presents a schematic illustrative example of a single-isocenter field setting for a patient;
FIG. 3 comprises a flow diagram as configured in accordance with various embodiments of these teachings;
FIG. 4 comprises a schematic representation as configured in accordance with various embodiments of these teachings;
FIG. 5 comprises a schematic representation as configured in accordance with various embodiments of these teachings;
FIG. 6 comprises a schematic representation as configured in accordance with various embodiments of these teachings; and
FIG. 7 comprises a schematic representation as configured in accordance with various embodiments of the invention.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, these teachings can be applied in conjunction with a radiation treatment platform having a corresponding platform isocenter and a movement capability for moving a patient with respect to the platform isocenter while undergoing radiation treatment by the radiation treatment platform. Generally speaking, these teachings can provide for a control circuit identifying a plurality of radiation treatment arclets for the radiation treatment platform, identifying, for each of the plurality of radiation treatment arclets, a corresponding isocenter location within the patient to thereby locate the platform isocenter at different locations within the patient during the course of a radiation treatment session, and optimizing a radiation treatment plan as a function of the plurality of radiation treatment arclets and their corresponding isocenter locations to provide an optimized radiation treatment plan.

By one approach, if desired, identifying the plurality of radiation treatment arclets can comprise automatically dividing at least one initial template arc into at least two radiation treatment arclets. These teachings will accommodate using arclets that are fully discrete from one another, or using at least some arclets that partially, but not wholly, overlap with one another.

By one approach, identifying the corresponding isocenter locations for each of the plurality of radiation treatment arclets can comprise identifying the corresponding isocenter locations to maintain close proximity of a radiation treatment platform collimator to an exterior surface of the patient. As one illustrative example in these regards, in the case where the radiation treatment plan comprises a stereotactic radiosurgery radiation treatment plan, the aforementioned exterior surface of the patient can comprise an exterior surface of the patient's head. (It may be noted that, by one approach, maintaining close proximity between the collimator and the exterior surface of the patient does not necessarily mean that the collimator is maintained in a close relationship with the treatment volume; instead, these teachings may be applied to achieve something more akin to the opposite result.)

By one approach, that close proximity can comprise, for example, no more than a predetermined collimator-to-skin distance. By one approach, identifying the plurality of radiation treatment arclets can further comprise determining arclet lengths for at least some of the radiation treatment arclets as a function of the aforementioned predetermined collimator-to-skin distance.

These teachings will then accommodate optionally administering therapeutic radiation to a patient via the aforementioned radiation treatment platform in a treatment session using the optimized radiation treatment plan. In such a case, if desired, these teachings will also accommodate automatically adjusting, during the treatment session, a distance between a surface of the patient (such as an exterior surface of the patient) and a part of the radiation treatment platform (such as a multi-leaf collimator) to avoid a collision therebetween.

So configured, these teachings can facilitate, for example, providing for multiple different isocenter positions that are automatically generated to maintain a relatively minimized distance between the multi-leaf collimator and the patient's skin to thereby reduce at least some unwanted effects of higher multi-leaf collimator elevations. In particular, these teachings can help to improve dose conformality.

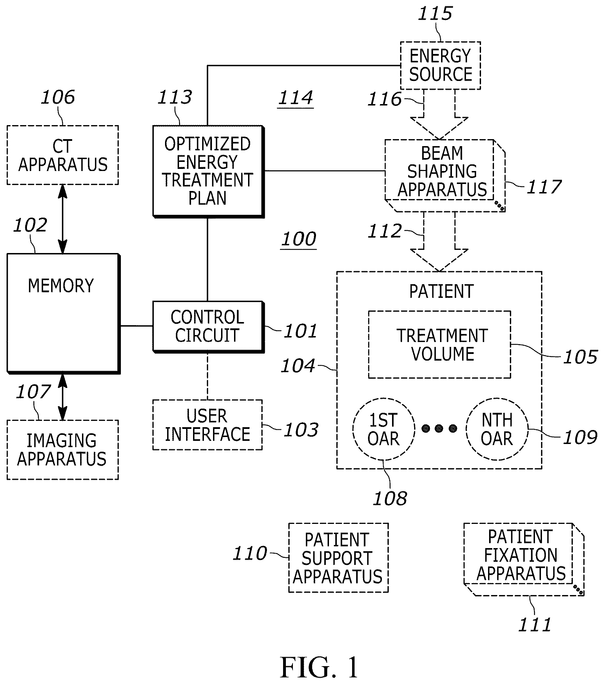

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is physically configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, the patient support apparatus 110 may be selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

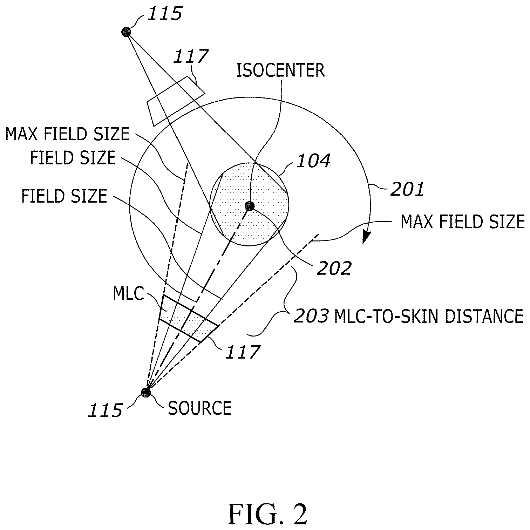

Before describing further aspects of these teachings in more detail, it may be helpful to the reader to explain and illustrate certain additional elements, features, and parameters of an illustrative radiation treatment application setting. FIG. 2 presents a schematic illustrative example of a single-isocenter field setting for a patient 104. For the sake of this example, it is presumed that this plan is for a stereotactic radiosurgery radiation treatment plan intended to administer therapeutic radiation to a tumor in the patient's head. Accordingly, this example also presumes that strict immobilization is used to prevent motion of the head during the treatment. The movement of the radiation source 115 in an arcuate path around the patient 104 is depicted by the circular arrow 201. Reference numeral 202 refers to the isocenter for the platform. Reference numeral 203 refers to the distance through air between the patient-facing side of the multi-leaf collimator (serving as the aforementioned beam shaping apparatus 117) and the outer surface of the patient's skin. (It will be noted that for head region treatments, it is often sufficient to use only a smaller central region (such as a 20 cm x 20 cm region (or even less)) of an otherwise available larger maximum field size (such as a 40 cm x 40 cm maximum field size).

In many typical application settings, the patient's tumor would be positioned coincident with the platform isocenter 202. So positioned, as the radiation source 115 rotates around the patient 104, the tumor will typically remain centrally located at the platform's isocenter.

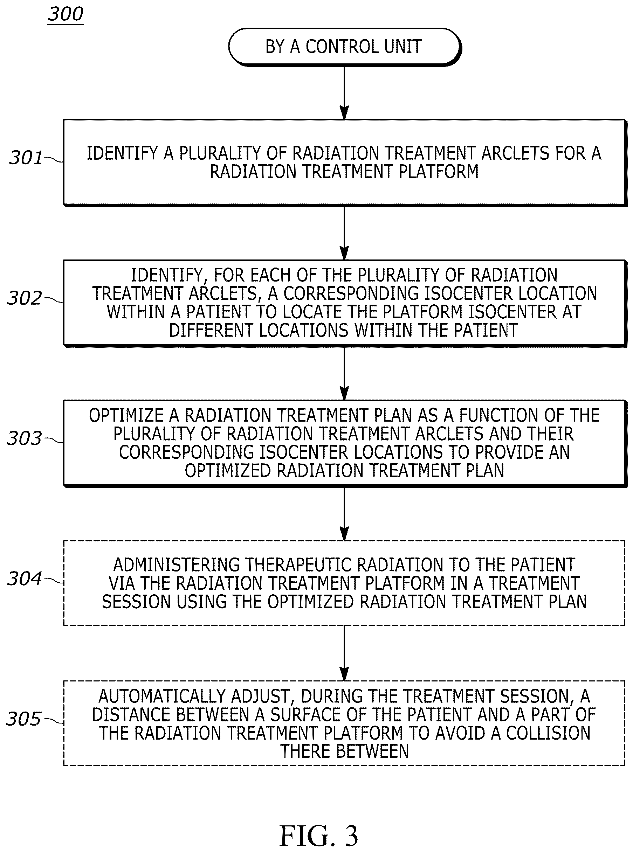

FIG. 3 presents a process 300 that can be carried out, for example, by the aforementioned control circuit 101. This process 300 can be used in conjunction with a radiation treatment platform 114 having a corresponding platform isocenter 202 and a movement capability of moving a patient with respect to the platform isocenter while undergoing radiation treatment by the radiation treatment platform. By one approach, the administration of radiation stops during such movement.

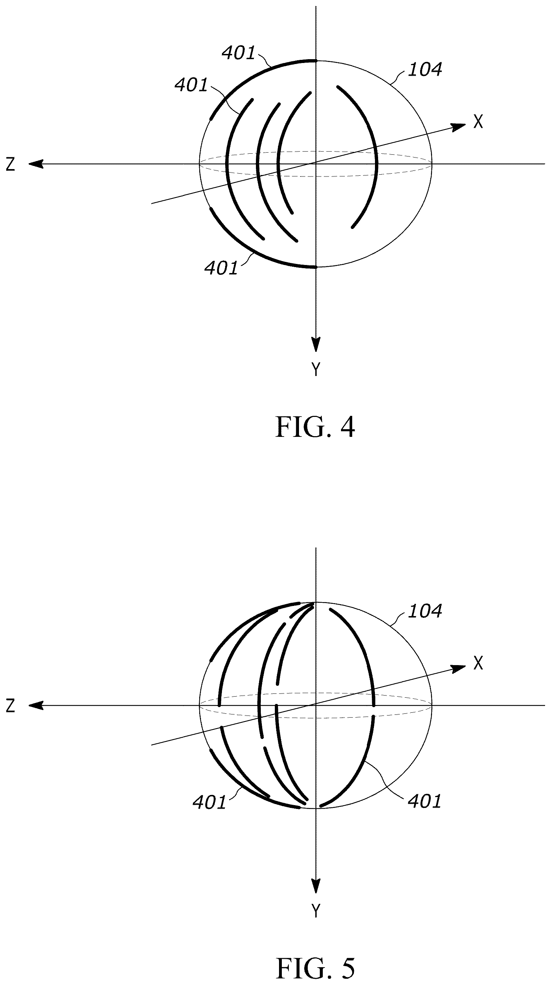

At block 301, the control circuit 101 identifies a plurality of radiation treatment arclets for the radiation treatment platform. An arclet is a partial segment of a continuous rotational therapy arc, where the radiation beam delivers treatment to a tumor from multiple angles along a partial or full circle around the patient. The present teachings will accommodate forming a plurality of arclets from a single partial/full circle around the patient, but will also accommodate forming at least one arclet from a plurality of differently-angled partial/full circles around the patient. FIG. 4 presents an illustrative example where six arclets are so identified (three of those arclets being denoted by reference numeral 401). FIG. 5 presents an illustrative example where eleven arclets 401 are so identified.

These teachings will accommodate having arclets that all share a same length, but for many application settings it may be preferable to at least accommodate arclet lengths that can vary. By one approach, for example, these teachings will accommodate determining arclet lengths for at least some of the radiation treatment arclets as a function of a predetermined collimator-to-skin distance (the latter being discussed below in more detail). For example, when the radiation treatment plan to be optimized is a stereotactic radiosurgery radiation treatment plan, the collimator-to-skin distance may be the distance between a multi-leaf collimator and the skin on the proximal (to the collimator) exterior surface of the patient's head.

By one approach, these teachings will accommodate having a clinician/technician identify some or all of these arclets. By another approach, these teachings will accommodate having the control circuit 101 automatically identify these arclets. As one illustrative example in the latter regards, the control circuit 101 may automatically divide at least one initial template arc into at least two radiation treatment arclets. One or more of an automatically created radiation treatment arc may then also be further subdivided, and so forth.

With continued reference to both FIGS. 4 and 5, these two examples may be viewed as using multiple static isocenter positioning templates. As exemplified in these examples, these teachings will accommodate taking into account that certain treatment directions might not be clinically favored (such as a direction coming directly from the top of the head, such that the radiation would travel through the patient's entire body all the way to the toes) or if there are regions where a standard template produces regions of densely placed arcs (such as, for example, a location in the forehead or in the back of the head). These examples could be used as templates, either determining entirely the field geometry, including the length of the arclets, and the exact positions of the isocenters, or they could describe just the center of each arclet with a corresponding possibly minimum acceptable arclet length and maximum allowed multi-leaf collimator-to-skin distance being specified, and then optimizing by, at least in part, balancing field coverage against the multi-leaf collimator-to-skin distance.

For many application settings, these arclets can all be separate and discrete from one another without any intersections. These teachings will also accommodate having at least two arclets that partially, but not wholly, overlap with one another.

At block 302, the control circuit 101 identifies, for each of the plurality of identified radiation treatment arclets, a corresponding isocenter location within the patient to thereby locate platform isocenters for different arclets at different locations within the patient. At least generally speaking, these isocenter locations within the patient are determined, for each corresponding arclet, so as to maintain close proximity of a radiation treatment platform collimator to an exterior surface of the patient (such as a most-proximal to the collimator exterior surface of the patient). By one approach, that close proximity is specified to be no more than a predetermined collimator-to-skin distance. That predetermined collimator-to-skin distance can vary with the needs and/or opportunities presented by a given application setting (including, but not limited to, the capabilities or limitations of a given radiation treatment platform and/or the specific presentation of a given patient). Examples of potentially useful distances include any value within a range of 1 cm to 25 cm, with a value within a range of 2 cm to 10 cm likely being beneficial for many application settings.

Generally speaking, in many cases these isocenter locations within the patient may be other than centrally located in the patient volume. In some cases, some or all of these isocenter locations within the patient may be disposed very close to an exterior surface of the patient.

At block 303, the control circuit 101 optimizes a radiation treatment plan as a function of the plurality of radiation treatment arclets and their corresponding isocenter locations within the patient to provide an optimized radiation treatment plan 113. In these regards, these teachings will accommodate using collimator angles that are determined by a template, or, optimizing collimator angles to seek an optimized best possible coverage of the treatment target structures by the multi-leaf collimator. The latter can even include using thinner leaves in a central region of the multi-leaf collimator.

At optional block 304, these teachings will then accommodate administering therapeutic radiation 112 to the patient 104 via the aforementioned radiation treatment platform 114 in a treatment session using the optimized radiation treatment plan 113. If desired, and as illustrated at optional block 305, these teachings will accommodate automatically adjusting, during the aforementioned treatment session, a distance between a surface of the patient 104 and a part of the radiation treatment platform 114 (such as the aforementioned multi-leaf collimator) to avoid a collision therebetween. Various dynamic collision avoidance approaches are known in the art and may be so applied in these regards. Employing a reliable and effective collision avoidance practice may permit using a smaller predetermined collimator-to-skin distance when identifying the isocenter locations within the patient for each arclet as described above.

Further details that comport with these teachings will now be presented. It will be understood that the specific details of these examples are intended to serve an illustrative purpose and are not intended to suggest any particular limitations with respect to these teachings.

These teachings can serve to automatically create arclets and to locate their corresponding isocenter locations within the patient so that those isocenter positions serve to reduce an average multi-leaf collimator-to-skin distance (or, in lieu of the foregoing or in combination therewith, a multi-leaf collimator-to-treatment-target distance).

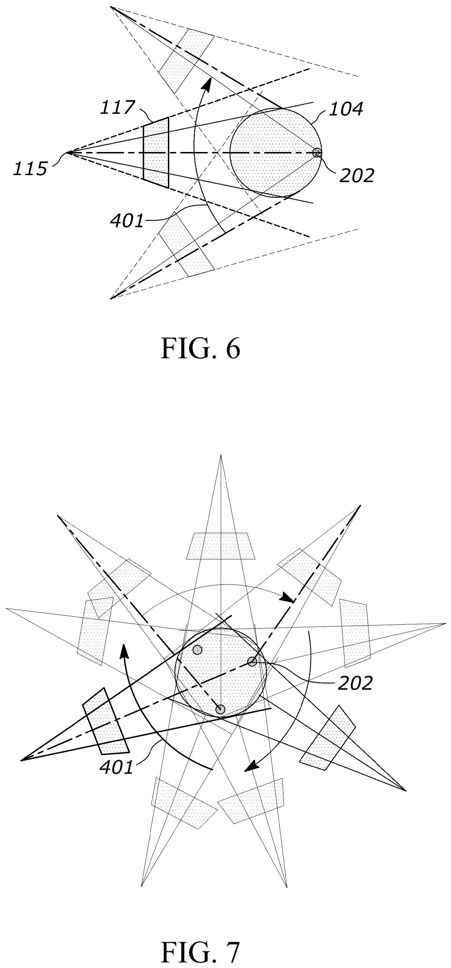

FIG. 6 presents an illustrative example in the foregoing regards. This figure, in particular, illustrates a simple case for a single less-than-half-arc arclet 401. The isocenter 202 for this arclet 401 is positioned so that it is away from the geometric center of the body (head) at a location that corresponds, at least approximately (say, within 1, 2, 3, 4 or 5 degrees) of the middle of the arclet 401. In this example, the isocenter 202 is located very near, though within, an exterior surface of the body (head).

As a result, the patient's body (head) is not necessarily coincident with or even near the center of the field. The applicant has determined that such a configuration can lead to some useful considerations regarding the arclet 401.

First, the further the location of the isocenter 202 from its traditional location at the center of the patient volume, the smaller the multi-leaf collimator-to-skin distance (and, accordingly, the narrower the penumbra of the beam). In addition, a shorter arclet can likely be utilized while simultaneously maintaining full coverage of the body (head) within the maximum available field size.

Second, because the dimensions of human heads do not greatly differ amongst a typical patient population, it can be generally pre-determined what would be a maximum allowed multi-leaf collimator-to-skin distance that would lead to an acceptable penumbra width without specifically taking into account the dimensions of a particular patient.

And third, the foregoing can help to inform the length of each arclet. The applicant notes that field coverage may often be increased by also using collimator angles that allow the use of a corner region of the field. It is also possible to only focus to a region where the treatment target(s) are located and to use the center of that region (rather than the center of the body) as a centroid of all such isocenter locations.

By one approach, immobilization of the radiation treatment platform 114 during the multiple isocenter location shifts can serve to help avoid risks that might otherwise be associated with intra-fraction patient movement.

The foregoing approaches, where the location of the body of the patient is shifted away from the centerline of the field, can impose a restriction on how large any given arclet can be when associated with a particular isocenter location. By one approach, and as illustrated in FIG. 7, to compensate for the foregoing, multiple different such arclets can be used to cover, in the aggregate, the same angular space that a single full arc would be expected to cover.

So configured, the foregoing use of a plurality of differently located isocenters within the patient, in correspondence with arclets, can result in improved dose conformality.

Further aspects of the invention are provided by the subject matter of the following clauses:
Clause 1. A method for use with a radiation treatment platform having a corresponding platform isocenter and a movement capability of moving a patient with respect to the platform isocenter while undergoing radiation treatment by the radiation treatment platform, the method comprising: by a control circuit: identifying a plurality of radiation treatment arclets for the radiation treatment platform; identifying, for each of the plurality of radiation treatment arclets, a corresponding isocenter location within the patient to locate the platform isocenter at different locations within the patient; optimizing a radiation treatment plan as a function of the plurality of radiation treatment arclets and their corresponding isocenter locations within the patient to provide an optimized radiation treatment plan.
Clause 2. The method of clause 1 wherein identifying the corresponding isocenter locations for each of the plurality of radiation treatment arclets comprises identifying the corresponding isocenter locations to maintain close proximity of a radiation treatment platform collimator to an exterior surface of the patient.
Clause 3. The method of either of clause 1 or 2 wherein the close proximity comprises no more than a predetermined collimator-to-skin distance.
Clause 4. The method of any of the foregoing clauses wherein identifying the plurality of radiation treatment arclets further comprises determining arclet lengths for at least some of the radiation treatment arclets as a function of the predetermined collimator-to-skin distance.
Clause 5. The method of any of the foregoing clauses wherein identifying the plurality of radiation treatment arclets comprises automatically dividing at least one initial template arc into at least two radiation treatment arclets.
Clause 6. The method of any of the foregoing clauses wherein the patient has a head and wherein the exterior surface of the patient comprises an exterior surface of the head.
Clause 7. The method of any of the foregoing clauses wherein the optimized radiation treatment plan comprises a stereotactic radiosurgery radiation treatment plan.
Clause 8. The method of any of the foregoing clauses wherein identifying the plurality of radiation treatment arclets for the radiation treatment platform comprises identifying at least two radiation treatment arclets that partially, but not wholly, overlap with one another.
Clause 9. The method of any of the foregoing clauses further comprising:
   administering therapeutic radiation to the patient via the radiation treatment platform in a treatment session using the optimized radiation treatment plan.
Clause 10. The method of any of the foregoing clauses further comprising:
   automatically adjusting, during the treatment session, a distance between a surface of the patient and a part of the radiation treatment platform to avoid a collision therebetween.
Clause 11. An apparatus for use with a radiation treatment platform having a corresponding platform isocenter and a movement capability of moving a patient with respect to the platform isocenter while undergoing radiation treatment by the radiation treatment platform, the apparatus comprising: a control circuit configured to: identify a plurality of radiation treatment arclets for the radiation treatment platform; identify, for each of the plurality of radiation treatment arclets, a corresponding isocenter location within the patient to locate the platform isocenter at different locations within the patient; optimize a radiation treatment plan as a function of the plurality of radiation treatment arclets and their corresponding isocenter locations to provide an optimized radiation treatment plan.
Clause 12. The apparatus of clause 11 wherein the control circuit is configured to identify the corresponding isocenter locations for each of the plurality of radiation treatment arclets by identifying the corresponding isocenter locations to maintain close proximity of a radiation treatment platform collimator to an exterior surface of the patient.
Clause 13. The apparatus of clause 12 wherein the close proximity comprises no more than a predetermined collimator-to-skin distance.
Clause 14. The apparatus of any of clauses 11 through 13 wherein the control circuit is further configured to identify the plurality of radiation treatment arclets by determining arclet lengths for at least some of the radiation treatment arclets as a function of the predetermined collimator-to-skin distance.
Clause 15. The apparatus of any of clauses 11 through 14 wherein the control circuit is configured to identify the plurality of radiation treatment arclets by automatically dividing at least one initial template arc into at least two radiation treatment arclets.
Clause 16. The apparatus of any of clauses 11 through 15 wherein the patient has a head and wherein the exterior surface of the patient comprises an exterior surface of the head.
Clause 17. The apparatus of any of clauses 11 through 16 wherein the optimized radiation treatment plan comprises a stereotactic radiosurgery radiation treatment plan.
Clause 18. The apparatus of any of clauses 11 through 17 wherein the control circuit is configured to identify the plurality of radiation treatment arclets for the radiation treatment platform by identifying at least two radiation treatment arclets that partially, but not wholly, overlap with one another.
Clause 19. The apparatus of any of clauses 11 through 18 wherein the control circuit is further configured to: administer therapeutic radiation to the patient via the radiation treatment platform in a treatment session using the optimized radiation treatment plan.
Clause 20. The apparatus of any of clauses 11 through 19 wherein the control circuit is further configured to: automatically adjust, during the treatment session, a distance between a surface of the patient and a part of the radiation treatment platform to avoid a collision therebetween.

Those skilled in the art will recognize that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A method for use with a radiation treatment platform having a corresponding platform isocenter and a movement capability of moving a patient with respect to the platform isocenter, the method comprising:
by a control circuit:
identifying a plurality of radiation treatment arclets for the radiation treatment platform;
identifying, for each of the plurality of radiation treatment arclets, a corresponding isocenter location within the patient to locate the platform isocenter at different locations within the patient;
optimizing a radiation treatment plan as a function of the plurality of radiation treatment arclets and their corresponding isocenter locations within the patient to provide an optimized radiation treatment plan.

2. The method of claim 1 wherein identifying the corresponding isocenter locations for each of the plurality of radiation treatment arclets comprises identifying the corresponding isocenter locations to maintain close proximity of a radiation treatment platform collimator to an exterior surface of the patient.

3. The method of claim 2 wherein the close proximity comprises no more than a predetermined collimator-to-skin distance.

4. The method of claim 3 wherein identifying the plurality of radiation treatment arclets further comprises determining arclet lengths for at least some of the radiation treatment arclets as a function of the predetermined collimator-to-skin distance.

5. The method of any one of claims 1 to 4 wherein identifying the plurality of radiation treatment arclets comprises automatically dividing at least one initial template arc into at least two radiation treatment arclets.

6. The method of any one of claims 1 to 5 wherein identifying the plurality of radiation treatment arclets for the radiation treatment platform comprises identifying at least two radiation treatment arclets that partially, but not wholly, overlap with one another.

7. An apparatus for use with a radiation treatment platform having a corresponding platform isocenter and a movement capability of moving a patient with respect to the platform isocenter while undergoing radiation treatment by the radiation treatment platform, the apparatus comprising:
a control circuit configured to:
identify a plurality of radiation treatment arclets for the radiation treatment platform;
identify, for each of the plurality of radiation treatment arclets, a corresponding isocenter location within the patient to locate the platform isocenter at different locations within the patient;
optimize a radiation treatment plan as a function of the plurality of radiation treatment arclets and their corresponding isocenter locations to provide an optimized radiation treatment plan.

8. The apparatus of claim 7 wherein the control circuit is configured to identify the corresponding isocenter locations for each of the plurality of radiation treatment arclets by identifying the corresponding isocenter locations to maintain close proximity of a radiation treatment platform collimator to an exterior surface of the patient.

9. The apparatus of claim 8 wherein the close proximity comprises no more than a predetermined collimator-to-skin distance, and, optionally, wherein the control circuit is further configured to identify the plurality of radiation treatment arclets by determining arclet lengths for at least some of the radiation treatment arclets as a function of the predetermined collimator-to-skin distance.

10. The apparatus of claim 7, 8 or 9 wherein the control circuit is configured to identify the plurality of radiation treatment arclets by automatically dividing at least one initial template arc into at least two radiation treatment arclets.

11. The apparatus of any one of claims 7 to 10 wherein the control circuit is configured to identify the plurality of radiation treatment arclets for the radiation treatment platform by identifying at least two radiation treatment arclets that partially, but not wholly, overlap with one another.

12. The apparatus of any one of claims 7 to 11 wherein the control circuit is further configured to:
administer therapeutic radiation to the patient via the radiation treatment platform in a treatment session using the optimized radiation treatment plan.

13. The apparatus of claim 12 wherein the control circuit is further configured to:
automatically adjust, during the treatment session, a distance between a surface of the patient and a part of the radiation treatment platform to avoid a collision therebetween.

14. The method of any one of claims 1 to 6 or the apparatus of any one of claims 7 to 13 wherein the patient has a head and wherein the exterior surface of the patient comprises an exterior surface of the head.

15. The method or apparatus of any one of the preceding claims wherein the optimized radiation treatment plan comprises a stereotactic radiosurgery radiation treatment plan.
